(19) Europäisches Patentamt
European Patent Office
Office européen des brevets

(11) EP 2 320 739 B1

(12) EUROPEAN PATENT SPECIFICATION

(45) Date of publication and mention of the grant of the patent:
27.11.2019 Bulletin 2019/48

(21) Application number: 09803270.9

(22) Date of filing: 30.07.2009

(51) Int Cl.:
A01N 43/90 (2006.01) A61K 31/519 (2006.01)

(86) International application number:
PCT/US2009/004399

(87) International publication number:
WO 2010/014234 (04.02.2010 Gazette 2010/05)

(54) **PHARMACEUTICAL COMPOSITIONS AND METHODS FOR STABILIZING THE SAME**

PHARMAZEUTISCHE ZUSAMMENSETZUNGEN UND VERFAHREN ZU IHRER STABILISIERUNG

COMPOSITIONS PHARMACEUTIQUES ET LEURS PROCÉDÉS DE STABILISATION

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **01.08.2008 US 85594 P**
**03.09.2008 US 93810 P**

(43) Date of publication of application:
**18.05.2011 Bulletin 2011/20**

(73) Proprietor: **Chiesi Farmaceutici S.p.A.**
**43122 Parma (IT)**

(72) Inventors:
- **MOTHERAM, Rajeshwar**
  **Dayton**
  **NJ 08810 (US)**
- **KRISHNA, Gopal**
  **Randolph**
  **NJ 07869 (US)**
- **MIN, Ding**
  **Tarrytown**
  **NY 10591 (US)**

(74) Representative: **Bianchetti Bracco Minoja S.r.l.**
**Via Plinio, 63**
**20129 Milano (IT)**

(56) References cited:
**EP-A1- 1 813 274**
**US-A- 4 150 744**
**US-A- 4 693 892**
**US-A- 5 739 152**
**US-A1- 2006 160 834**

- **GYLLENHAAL: "Packed-column supercritical fluid chromatography for the purity analysis of clevidipine, a new dihydropyridine drug", JOURNAL OF CHROMATOGRAPHY, vol. 862, no. 1, 1999, pages 95-104, XP009150407,**
- **JAREMO ET AL.: 'Supercritical Fluid Extraction of Clevidipine from a Water Based Vegetable Oil Emulsion.' JOUMAL OF LIQUID CHROMATOGRAPHY & RELATED TECHNOLOGIES vol. 21, no. 3, 1998, pages 391 - 406, XP009150421**
- **Kapitel 4: "Physikalisch-chemische Grundlagen für Arzneiformen" In: Bauer Frömming Führer: "Lehrbuch der Pharmazeutischen Technologie", 1999, Wissenschaftliche Verlagsgesellschaft mbH Stuttgart ISBN: 3-8047-1700-4 vol. 6 * Pages 96 and 98 ***

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 320 739 B1

## Description

### Field of the Invention

The instant invention relates to a process for preparing stabilized pharmaceutical compositions, having clevidipine as an active ingredient, with minimized levels of impurities.

### Background of the Invention

Clevidipine, which is also known as Cleviprex™, is a short-acting, vascular selective calcium antagonist that has been shown to reduce arterial blood pressure with a fast termination of effect due to metabolism by blood and tissue esterases. As an arterial-selective vasodilator, clevidipine reduces peripheral vascular resistance directly, without dilating the venous capacitance bed.

The chemical name of clevidipine is butyroxymethyl methyl 4-(2',3'-dichlorophenyl)-1,4-dihydro-2,6-dimethyl-3,5-pyridinedicarboxylate ($C_{21}H_{23}Cl_2NO_6$). Its corresponding structure is as follows:

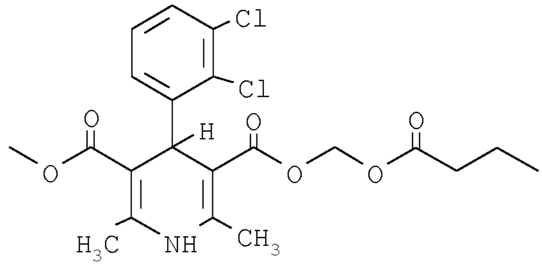

Clevidipine is typically formulated as a liquid emulsion suitable for intravenous administration. Lipid emulsions are widely used in parenteral nutrition use for approximately 30 years and in the recent past have been used as drug carriers for insoluble drugs such as propofol (Diprivan®), and diazepam. Apart from their ability to deliver insoluble drugs, emulsions are also suitable dosage forms for drugs like clevidipine that are susceptible to hydrolytic breakdown. Emulsions have also been reported to prevent drugs from adhering to plastic administration sets used during intravenous injection, and reduce local toxicity on infusion.

As a pharmaceutical composition, it is essential that clevidipine maintains its stability. Over the past several years, various impurities have been identified in compositions containing clevidipine as an active ingredient. For example, some impurities arise from the process used in making clevidipine, while others are due to gradual degradation of the active ingredient. As a pharmaceutical composition, it is essential to maintain stability and minimize the amount of impurities regardless of their source or the mechanism of degradation.

Therefore, a need exists for methods for maintaining the stability of compositions having clevidipine as an active ingredient. There also exists a need for compositions of clevidipine having acceptable stability profiles with respect to their ultimate potency and impurity levels.

US5739152 discloses emulsions comprising clevidipine, egg lecithin, glycerol and water for injection. A method for manufacturing these emulsion is also disclosed in general terms.

### Summary of the Invention

The present invention draws upon the discovery that certain impurities in a pharmaceutical composition comprising clevidipine can be minimized by reducing or preventing an oxidation reaction.

More specifically, the present invention draws upon the discoveries that a pharmaceutical composition having clevidipine as an active ingredient is sensitive to light exposure and exposure to air. Upon exposure to light, clevidipine is prone to degradation. Light exposure may give rise to an unacceptable level of an impurity, known as H324/78, which is also called butyroxylmethyl methyl 4-(2',3'-dichlorophenyl)-2,6-dimethyl-3,5-pyridinedicarboxylate). Its structure is shown in the following formula:

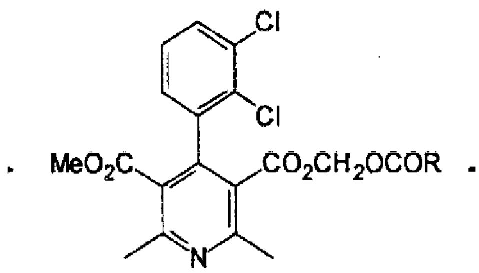

**H324/78**
$R = CH_2CH_2CH_3$

Accordingly, the first aspect of the present invention describes a process for preparing a pharmaceutical composition that is stabilised against oxidation, wherein the composition is an emulsion for intravenous administration comprising:

0.5 mg per mL of clevidipine or a pharmaceutically acceptable salt thereof,
20% soybean oil,
glycerin,
purified egg yolk phospholipids,
water,
and sodium hydroxide,
wherein the process comprises the steps of:

(a) dispensing water for injection to a mix tank at 74 °C to 78 °C;
(b) adding glycerin to the water and cooling the resulting aqueous phase to 60 °C to 70 °C;
(c) dispensing soybean oil into a dissolving tank to form an oil phase;
(d) mixing and heating the soybean oil to 70 °C to 82 °C;
(e) adding clevidipine to the soybean oil mixture and heating to 78 °C to 82 °C;
(f) adding egg yolk phospholipids to the mixture of clevidipine and soybean oil;
(g) mixing the aqueous phase and the oil phase to form an emulsion;
(h) adjusting the pH with 1N sodium hydroxide to a pH of 6.0 to 8.8; and
(i) homogenising the emulsion at a pressure of 500/8000psi at a temperature of 50 °C to 55 °C;, and wherein oxidation of the composition is minimised by reducing the amount of light exposure during the manufacturing and storing processes, such that the amount of H324/78 having the following formula:

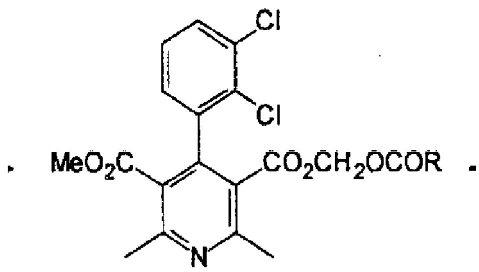

**H324/78**
$R = CH_2CH_2CH_3$

is less than or equal to about 0.2% on a weight-to-weight basis to clevidipine.

The process includes the slowing down or inhibiting of the oxidation pathway of clevidipine. This is accomplished by reducing the amount the pharmaceutical composition is exposed to light during the manufacturing and storing processes, such that these processes do not need to be performed under any sort of special or altered lighting conditions.

According to this process, light can be sufficiently blocked such that light energy cannot reach the active ingredient of the composition, or is reduced to a level that the light-induced oxidation reaction converting clevidipine to H324/78 is minimized, such that the total detectable level of H324/78 in a given composition sample does not exceed about 0.2% on a weight-by-weight basis, or the ratio of clevidipine to H324/78 on a weight-to-weight basis is equal or greater than about 450 to 1.

The present invention further includes a process for preparing a pharmaceutical composition having clevidipine as an active ingredient, comprising placing the composition in a sealable container that reduces the amount clevidipine is exposed to light, such that the level of H324/78 does not exceed 0.2% on a weight-to-weight basis. The container can be tinted, pigmented, colored, opaque or other dark color or material. The method can also include placing the container into a secondary packaging that further reduces the amount clevidipine is exposed to light. The secondary packaging can be a carton, wrap, or other forms of secondary covering.

The present invention also includes a process for preparing a pharmaceutical composition having clevidipine as the active ingredient, including the slowing down or otherwise inhibiting of the oxidation pathway of clevidipine by reducing or inhibiting the amount of oxygen in the process of manufacturing and/or storing the composition. This can be done by replacing oxygen with nitrogen. The same can be done in the emulsification process, where the components are blanketed with an inert gas, such as nitrogen, for example, throughout the process of manufacturing to minimize and/or replace oxidizing compounds.

A method of identifying and quantifying levels of H324/78 in pharmaceutical samples having clevidipine as an active ingredient can be accomplished by column chromatography, such as high pressure liquid chromatography ("HPLC"). Based on this method of detecting H324/78, the lower limit of detection, or the minimum detectible level of H324/78, may be approximately 0.01% of the clevidipine active ingredient. Alternatively, the lower limit of H324/78 detection can be set forth as a ratio of clevidipine to H324/78, where the ratio of the HPLC peak areas of clevidipine to H324/78 can be as high as 10,000 to 1.

## Brief Description of the Figures

Understanding of the present invention will be facilitated by consideration of the following detailed description of the embodiments of the present invention taken in conjunction with the accompanying drawings, in which like numerals refer to like parts and in which:

FIG. 1 illustrates a proposed degradation pathway of clevidipine;

FIG. 2A is an HPLC chromatogram illustrating the level of detection of the various clevidipine degradants in Sample 2;

FIG. 2B is an HPLC chromatogram illustrating the level of detection of the various clevidipine degradants in Sample 3; and

FIG. 3 illustrates an overlay of HPLC Chromatograms of various peaks relating to clevidipine and various other compounds.

## Detailed Description of the Preferred Embodiments

It is to be understood that the figures and descriptions of the present invention have been simplified to illustrate elements that are relevant for a clear understanding of the present invention, while eliminating, for the purpose of clarity, many other elements found in typical pharmaceutical compositions and methods of stabilization. Those of ordinary skill in the art will recognize that other elements and/or steps are desirable and/or required in implementing the present invention. However, because such elements and steps are well known in the art, and because they do not facilitate a better understanding of the present invention, a discussion of such elements and steps is not provided herein. The disclosure herein is directed to all such variations and modifications to such elements and methods known to those skilled in the art.

As mentioned previously, clevidipine is a fast acting dihydropyridine calcium channel blocking agent developed for the treatment of various conditions, such as hypertension, including primary hypertension, secondary hypertension, acute hypertension, chronic hypertension and perioperative hypertension in cardiac surgery, high blood pressure, chest pain (angina), migraines, brain aneurysm complications, irregular heartbeats (arrhythmia) and Raynaud's disease. As an arterial-selective vasodilator, clevidipine reduces peripheral vascular resistance directly, without dilating the venous capacitance bed. The end effect can be a reduction in systolic blood pressure.

As used herein, the term "clevidipine" shall mean and include all varieties of forms of clevidipine. Unless otherwise specified, examples of such forms include all pharmaceutically acceptably salts, esters, isomers, stereoisomers, crystalline and amorphous forms.

As used herein, the term "pharmaceutically acceptable salt" shall refer to salts prepared from pharmaceutically acceptable non-toxic bases or acids including inorganic or organic bases and inorganic or organic acids. Examples of salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like.

The present invention draws upon the discovery that a pharmaceutical composition having clevidipine as an active ingredient is sensitive to degradation under certain conditions. More specifically, it was discovered that light exposure and exposure to oxygen lead to an accelerated degradation of clevidipine, and such degradation can compromise the purity and ultimately the potency of clevidipine. As shown in the flow diagram of Figure 1, the degradation pathway for clevidipine includes a number of chemical processes and can lead to numerous impurities, such as H324/78, H152/66, H152/81, H168/79, H207/59, and H207/36, for example.

It has been discovered that, under light, clevidipine oxidizes into H324/78, the pyridine analog of the active ingredient. H324/78 is also called Butyroxylmethyl methyl 4-(2',3'-dichlorophenyl)-2,6-dimethyl-3,5-pyridinedicarboxylate, and is shown in the following formula:

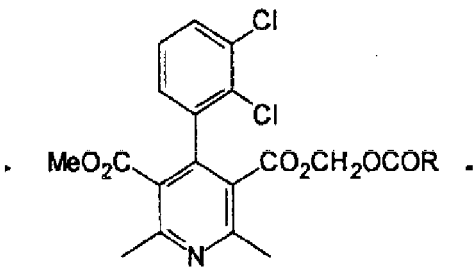

**H324/78**

$R = CH_2CH_2CH_3$

Oxidation of clevidipine can be slowed by reducing the amount of light exposure to the pharmaceutical composition during the manufacturing and storing process, such that these processes do not need to be performed under any sort of special or altered lighting conditions. To accomplish this, light must be sufficiently blocked such that light energy cannot reach the active ingredient of the composition, or is reduced to a level that the light-induced oxidation reaction converting clevidipine to H324/78 is minimized, such that the total level of H324/78 in a given composition sample does not exceed about 0.2%, or the ratio of clevidipine to H324/78 does not exceed about 500 to 1.

In one exemplary embodiment of the present invention, clevidipine emulsions are stored in sealable and tinted, pigmented, colored, opaque or otherwise dark, primary containers (such as bottles, bags or tubes, for example), as understood by those skilled in the art to reduce the amount of light exposure to the contained materials. For example, dark colored glass bottles can be used, pigmented high-density polyethylene ("HDPE") containers can be used, or any other sort of light blocking container, provided the container is pharmaceutical grade and does not contaminate the pharmaceutical composition contained therein.

In another embodiment, the process may include the steps of placing the primary container into a secondary packaging, such as a carton, box, wrap, or other container or covering suitable for reducing the amount of light exposure to the primary container. For example, such secondary packaging can include an aluminum wrap or overseal. A secondary HDPE container may also be used. The secondary packaging may also be tinted, pigmented, colored, opaque or otherwise dark to prevent or reduce the amount of light exposure to the active ingredient of the pharmaceutical composition.

The secondary packaging can be used in conjunction with the step of providing a protective primary container, or it may be used with a standard, unprotective primary container. For example, the process may include the storing of the pharmaceutical composition containing clevidipine as an active ingredient in a sealed, clear glass primary container, and is further covered with an aluminum overseal. In a further exemplary embodiment, these covered glass containers

may be packaged in cartons that further reduce light exposure. As described herein, any number or combination of protective and non-protective primary and secondary containers or packaging may be used.

Clevidipine is manufactured by reaction of 4-(2',3'-dichlorophenyl)-1,4-dihydro-5-methoxycarbonyl-2,6-dimethyl-3-pyridinecarboxylic acid with chloromethyl butyrate to obtain clevidipine. This reaction can be done optionally in the presence of a corresponding hydrogen carbonate, such as $KHCO_3$, in refluxing acetonitrile. Inorganic salts can be removed by filtration and the product is crystallized by the addition of isopropanol and water with subsequent cooling. It can also be crystallized by exchanging solvent from acetonitrile to a mixture of alcohol, such as ethanol or isopropanol, and water with repeated evaporations. In the further purification of the product, the crystals are washed with a mixture of water and ethanol or isopropanol. The product can be dissolved in refluxing isopropanol, crystallized by cooling, isolated by filtration and finally washed with a water and isopropanol mixture. A more detailed description of the manufacturing process of clevidipine can be found in U.S. Patent No. 6,350,877.

Clevidipine is typically formulated as a liquid emulsion suitable for intravenous administration. Lipid emulsions are widely used in parenteral nutrition use for approximately 30 years and in the recent past have been used as drug carriers for insoluble drugs such as propofol (Diprivan®), and diazepam. Apart from their ability to deliver insoluble drugs, emulsions are also suitable dosage forms for drugs like clevidipine that are susceptible to hydrolytic breakdown. Emulsions have also been reported to prevent drugs from adhering to plastic administration sets used during intravenous injection, and reduce local toxicity on infusion.

Typically, each mL may contain 0.5 mg clevidipine in approximately 20% soybean oil emulsion for intravenous administration. Other ingredients may include glycerin, purified egg yolk phospholipids and sodium hydroxide to adjust pH. Generally, water for injection is dispensed to a mix tank at 74°C - 78°C. Glycerin is added, and the aqueous phase is cooled to 60°C - 70°C prior to addition of the oil phase. For the oil phase, soybean oil is dispensed into a dissolving tank, mixed and heated to 70°C - 82°C. Clevidipine is then added to the soybean oil mixture and heated to 78°C - 82°C. Egg yolk phospholipids are then added to the mixture. The aqueous and oil phases are mixed together to form an emulsion, and the pH is adjusted with 1N sodium hydroxide to a pH of 6.0 to 8.8. The emulsion is then homogenized at a pressure of 500/8000 psi and a temperature of 50°C - 55°C to a fine particle size. The samples are filtered and dispensed into 50mL or 100mL bottles and capped with siliconized rubber stoppers, and crimp sealed with an aluminum overseal. Further information regarding the formulation of clevidipine can be found in U.S. Patent No. 5,739,152..

According to an aspect of the present invention, the above described process may include the removal of oxygen from either or both of the manufacturing and storing processes. For example, this can be done by replacing oxygen an inert gas, such as nitrogen. The same can be done in the emulsification process, where the components are blanketed with an inert gas, such as nitrogen, throughout the process to minimize and/or replace oxidizing compounds.

The above mentioned methods of stabilizing pharmaceutical compounds having clevidipine as an active ingredient provide a shelf life of at least 36 months for the compositions, when stored at 2°C to 8°C. After being removed from this refrigerated condition and placed at roughly room temperature (15°C to 30°C), the compositions remain stable for up to at least 2 additional months.

A method of detecting H324/78 in pharmaceutical samples having clevidipine as an active ingredient includes the step of isolating the individual chemical compounds making up the degradants or impurities found in the clevidipine degradation pathway. This can be accomplished by column chromatography, such as high pressure liquid chromatography ("HPLC"), for example. The pharmaceutical sample having clevidipine as an active ingredient can be introduced in small volume to the column and the resulting analysis of the eluent may illustrate the isolation and identification of peaks representative of the degradants, such as H324/78. As may be understood by those skilled in the art, any optimization of the HPLC method may be performed to give the best separation of peaks as between the various impurities found in the degradation of clevidipine. Examples of such detection can be seen in Figure 2, where the detection of various degradants within clevidipine emulsion samples was made by HPLC. Other methods of detecting may also be used, such as nuclear magnetic resonance spectroscopy, or other spectroscopic techniques as understood by those skilled in the art.

Based on these methods of detecting H324/78, the lower limit of detection, or the minimum detectible level of H324/78, may be approximately 0.01% of the pharmaceutical composition containing clevidipine as an active ingredient. Alternatively, the lower limit of H324/78 detection may be set forth as a ratio of clevidipine to H324/78, where the ratio of the HPLC peak areas of clevidipine to H324/78 may be as high as 10,000 to 1. However, it should be understood that because other methods of detection may be used, these other methods may enable detection of H324/78, or any other degradant or impurity, at even lower limits.

The present invention further includes pharmaceutical compositions having clevidipine and any of its pharmaceutically acceptable salts, as an active ingredient, wherein the pharmaceutical composition is stored or prepared so as to minimize oxidation degradation.

The pharmaceutical compositions prepared by the process of the invention have an effective amount of clevidipine, and any of its pharmaceutically acceptable salts, as an active ingredient, wherein the amount of H324/78 is equal or no greater than 0.2% on a weight-by-weight basis. For example, such pharmaceutical compositions may include an

amount of clevidipine ranging between 90% to 99.99%, and an amount of H324/78 ranging between 0.01% to 0.2% on a weight-to-weight basis to clevidipine. These compositions may further include any excipients as understood by those skilled in the art, as well as other degradants in variable amounts as described herein, provided the required level of potency of clevidipine remains sufficient and effective for use to treat any indication as described herein. The amount of clevidipine may be 90%, to 110%. The amount of H324/78 can be equal or no greater than about0.2% and preferably 0.1% and most preferably 0.05% on a weight-to-weight basis, for example.

The pharmaceutical composition prepared by the process of the invention includes clevidipine as an active ingredient and H324/78, where the ratio of the HPLC peak areas between clevidipine and H324/78 is between 500 to 1 and 10,000 to 1. In more particular embodiments of the pharmaceutical composition of the present invention, the ratio between clevidipine and H324/78 can be 1000 to 1, and 2000 to 1. As mentioned previously, these compositions may further include any excipients as understood by those skilled in the art, as well as other degradants in variable amounts as described herein, provided the required level of potency of clevidipine remains sufficient and effective for use to treat any indication as described herein.

As shown in the following examples, impurity levels were evaluated to determine the stability of clevidipine emulsions contained in various packaging materials for their ability to minimize degradation of clevidipine under exposure to light.

As described herein, the various pharmaceutical compositions prepared by the process of the present invention may be used for treating or preventing a disease or condition. As used herein, a disease or condition shall refer to a disease or condition which may be treated or prevented by a selective calcium channel blocker. Examples of such disease or condition include, without limitation, hypertension, such as primary hypertension, secondary hypertension, acute hypertension, chronic hypertension, high blood pressure, chest pain (angina), migraines, brain aneurysm complications, irregular heartbeats (arrhythmia) and Raynaud's disease. The compositions can be formulated as an emulsion and administered intravenously by injection.

Example 1 (Photostability study)

In a first study, 0.5 mg/mL clevidipine emulsion samples were packaged in 100mL type II bottles and sealed with black bromobutyl stoppers.

Table I - Sample Description for Photostability Study

| Sample ID | No. of bottles | Sample Description | Secondary Packaging | Light Exposure Condition |
|---|---|---|---|---|
| Sample 1 | 16 | Bottles without secondary packaging | None | None, Stored in 5 ± 3 °C/ambient humidity |
| Sample 2 | 16 | Bottles which are wrapped in aluminum foil | None | ICH light requirements 25 ± 2 °C/60 ± 5% RH |
| Sample 3 | 16 | Bottles without secondary packaging | None | ICH light requirements 25 ± 2 °C/50 ± 5% RH |
| Sample 4 | 16 | Bottles with secondary packaging | Cardboard carton | ICH light requirements 25 ± 2 °C/60 ± 5% RH |

As shown in Table 1, these samples were placed into four equal groups, Identified as samples 1-4. Sample 1 served as a control and was not to be exposed to light. Sample 2 was wrapped in foil and was to be exposed to light. Sample 3 was the bottled clevidipine emulsion and was to be exposed to light. Sample 4 was the bottled clevidipine emulsion contained in secondary packaging consisting of individual plain, white cartons and was to be exposed to light.

Samples 2-4 were exposed to light in a light chamber maintained at 25 ± 2°C/60% ± 5% RH and fitted with cool white fluorescence lights and near ultraviolet light. This exposure provided an overall illumination of about 1.32 million lux hours and an integrated near ultraviolet energy of about 220 watt hours/square meter. After exposure to light to the appropriate sample groups, all samples were analyzed by HPLC. An example of the detection of the various degradants by HPLC in Samples 2 and 3 are illustrated in Figure 2.

As shown in Table 2, there was an increase in the H324/78 degradation peak in Sample 3, equaling a degradant of about 0.3%, as compared to degradant levels of about 0.1% for Samples 1, 2 and 4.

TABLE II - Results of Photostability Study in Secondary Packaging for Clevidipine Emulsion

| Test / Test Method | | Acceptance Criteria | With Light Exposure at 25 ± 2 °C/ 60 ± 5 RH | | | 5 ± 3 °C/ Ambient Humidity |
|---|---|---|---|---|---|---|
| | | | Sample 4 | Sample 3 | Sample 2 | Sample 1 |
| Appearance/ QAC 3-0017 | | Pass | Pass | Pass | Pass | Pass |
| pH/QAC 3-0017 | | 6.0 - 8.9 | 7.5 | 7.5 | 7.5 | 7.6 |
| Drop Size Distribution / QAC 2-0195 | | NMT 0.7μ | 0.4 | 0.4 | 0.4 | 0.4 |
| | | NMT 4% of droplets: > 1μ | 0 | 0 | 0 | 0 |
| | | 100% of Droplets: ≤ 1μ | 1 | 1 | 1 | 1 |
| Particulate Matter in Injections / QAC 2-0023 | | ≤ 10 μm: NMT 3000 particulates per container | 11 | 6 | 15 | 8 |
| | | ≤ 25 μm: NMT 300 particulates per container | 2 | 3 | 6 | 4 |
| Clevidipine Identification / QAC 2-0191 | | To pass Test | Pass | Pass | Pass | Pass |
| Clevidipine Assay / QAC 2-0191 | | 90-110% of label claim | 102 | 101 | 101 | 101 |
| Degradation products / QAC 2-0191 | H152/81 | NMT 4.0% | 0.4 | 0.3 | 0.4 | 0.5 |
| | H207/59 | NMT .0.5% | 0.0 | 0.0 | 0.0 | 0.0 |
| | H168/79 | NMT 2.0% | 0.4 | 0.4 | 0.5 | 0.4 |
| | H324/78 | Report % Area | 0.1 | 0.3 | 0.1 | 0.1 |
| | Other[1] | Report % Area | 0.8 | 0.8 | 0.8 | 0.8 |
| Total Degradation Products (%) / QAC 2-0191 | | NMT 5.0% | 1.7 | 1.8 | 1.8 | 1.8 |
| [1]Total area % of all unspecified peaks | | | | | | |

Example 2 (Photostability study)

In a second photostability study, two batches of twenty 0.5 mg/mL clevidipine emulsion samples were packaged in 50mL type I bottles and sealed with black bromobutyl stoppers.

Table 3 - Photostability Samples

| **Description** | **Total Luminous Intensity (Lux Hrs.)** | **Sample 1** | **Sample 2** | **Sample 3** | **Sample** 4 |
|---|---|---|---|---|---|
| 7 day Exposure | 1.32 million | - | 2 | 2 | 2 |
| 4 day Exposure | 0.75 million | - | 2 | 2 | 2 |
| 2 day Exposure | 0.38 million | - | 2 | 2 | 2 |
| Unexposed | - | 2 | | | |
| Sample per type | - | | 6 | 6 | 6 |
| Total samples for study | | 20 | | | |

As shown in Table 3, these samples were placed into four groups, identified as Samples 1-4. Sample 1 served as a control and was not to be exposed to light. Sample 2 was wrapped in aluminum foil and was to be exposed to light. Sample 3 was the bottled clevidipine emulsion samples and was to be exposed to light. Sample 4 was the bottled clevidipine emulsion samples contained in secondary packaging consisting of individual plain, white cartons and were

to be exposed to light.

Samples 2-4 were exposed to light in a light chamber maintained at 25 ± 2°C/40% RH and fitted with cool white fluorescence lights and near ultraviolet light. Samples 2-4 were exposed to light up to a period of 7 days along with intermediary exposure periods of 2 and 4 days. The total luminous intensity was calculated to be 380,000, 750,000 and 1,320,000 for 2, 4 and 7 days, respectively. Integrated near ultraviolet energy was calculated to be 220 watt hours/square meter. After exposure to light per Table 3, all samples were analyzed by HPLC. The same tests were run for both batches of clevidipine emulsion samples.

As shown in Tables 4 and 5, the level of potency of clevidipine and the amount of impurities in Samples 2 and 4 remained relatively unchanged in both batches for the 7 day period. In Sample 3, the level of potency of clevidipine and the amount of impurities, with the exception of H324/78, remained relatively unchanged in both batches for the 7 day period. In the first batch, the level of H324/78 increased, and exceeded 0.2% after 4 days of light exposure. However, H324/78 levels remained relatively unchanged after day 4 and through the remainder of the 7 day period. In the second batch, H324/78 levels increased but did not exceed 0.2% throughout the 7 day period.

Table IV: Photostability results of Clevidipine Emulsion (0.5) mg/mL)

| | | | | | | | | | | |
|---|---|---|---|---|---|---|---|---|---|---|
| Specifications 1 | 90-110% | NMT 4.0% | NMT 2.0% | NMT 0.5% | F10 | F10 | F10 | F10 | F10 | NMT 5.0% |
| Specifications 2 | 90-110% | NMT 1.5% | NMT 1.5% | NMT 0.7% | NMT 0.2% | NMT 0.2% | NMT 0.2% | NMT 0.2% | NMT 0.2% | NMT 2.5% |
| Sample 1 | 101 | 0.7 | 0.3 | <0.1 | 0.1 | ND | ND | <0.1 | ND | 1.23 |
| Sample 2 Day 7 | 101 | 0.6 | 0.5 | <0.1 | 0.1 | ND | ND | <0.1 | ND | 1.3 |
| Sample 4 Day 7 | 102 | 0.6 | 0.5 | <0.1 | 0.1 | ND | ND | <0.1 | ND | 1.3 |
| Sample 2 Day 2 | 103 | 0.4 | 0.4 | <0.1 | 0.2 | ND | ND | ND | 0.1 | 1.2 |
| Sample 2 Day 4 | 102 | 0.3 | 0.3 | <0.1 | 0.3 | ND | ND | ND | 0.2 | 1.2 |
| Sample 2 Day 7 | 100 | 0.3 | 0.4 | <0.1 | 0.3 | 0.2 | ND | ND | 0.3 | 1.6 |

**Table V: Photostability results of Clevidipine Emulsion (0.5) mg/mL)**

| | | | | | | | | | | |
|---|---|---|---|---|---|---|---|---|---|---|
| Specifications 1 | 90-110% | NMT 4.0% | NMT 2.0% | NMT 0.5% | NMT 0.2% | F10 | F10 | F10 | F10 | NMT 5.0% |
| Specifications 2 | 90 - 110% | NMT 1.5% | NMT 1.5% | NMT 0.7% | NMT 0.2% | NMT 0.2% | NMT 0.2% | NMT 0.2% | NMT 0.2% | NMT 2.5% |
| Sample 1 | 98 | 1.2 | 0.4 | <0.1 | 0.1 | ND | ND | ND | ND | 1.8 |
| Sample 2 Day 7 | 98 | 1.1 | 0.6 | <0.1 | 0.1 | ND | ND | ND | ND | 1.8 |
| Sample 4 Day 7 | 98 | 1.0 | 0.6 | <0.1 | 0.1 | ND | ND | ND | ND | 1.7 |
| Sample 2 Day 2 | 98 | 0.7 | 0.4 | <0.1 | 0.2 | ND | ND | <0.1 | 0.1 | 1.5 |
| Sample 2 Day 4 | 98 | 0.7 | 0.4 | <0.1 | 0.2 | ND | ND | <0.1 | 0.2 | 1.6 |
| Sample 2 Day 7 | 98 | 0.6 | 0.5 | <0.1 | 0.2 | 0.1 | ND | ND | 0.4 | 2.0 |

As shown in the preceding studies, levels of H324/78 increased when clevidipine emulsion samples were

exposed to light. Additionally, as shown in the second study, a new degradation product in Sample 3 was discovered. It was formed initially at about 0.1% after 2 days of light exposure, and was found to increase to 0.35% and 0.38% after 7 days of light exposure in the first and second batches, respectively. The increase in the new degradant occurred with the concomitant decrease in H152/81, which is the de-esterified analog of clevidipine. Thus, the new degradant was suspected to be H152/66, which is a pyridine analog resulting from the oxidation of the dihydropyridine H152/81. Referring back to the degradation pathway illustrated in Figure 1, H152/66 can be alternatively prepared by the base hydrolysis of H324/78. All samples, after exposure to light, retained the required or otherwise necessary potency of clevidipine to treat any of the indications as described herein.

To show that the structure of the new degradant was in fact H152/66, a sample of H324/78 was hydrolyzed by sodium hydroxide. The H324/78 hydrolysis sample was analyzed by HPLC and compared to the HPLC profile of the new degradant from the second study. An overlay of the HPLC chromatogram of H324/78, its hydrolysis sample, and the new degradant are shown in Figure 3. As shown in Figure 3, the new degradant elutes at the same retention time as that of the base hydrolysis product of H324/78, and therefore confirms that the new degradation product formed was H152/66.

Typical HPLC methods useful in the present invention are presented in Examples 3 and 4.

Example 1 HPLC Procedure

Clevidipine assay and related substances were tested at each time point by a stability indicating method. This method is an isocratic, normal phase HPLC method with peak detection at 220 nm wavelength.

Column temperature: 35-40 degrees C.
Injection volume: 20 μl.
Flow rate: 1.0 ml/min.
Run time about 25 minutes.

Mobile phase of Heptane:ethanol (90:10) is employed and used for the assay of clevidipine and the degradation products with the exception of Substance 24.

Condition column with clevidipine mobile phase at 1.0mL/min for 4 hours.

New column should be conditioned overnight at 0.2mL/min.

When a degradation product is eluted, column can be washed with filtered ethanol for about 2 hours at 1.0 mL/min, then proceed with equilibration.

Examples of Column: PVA silica column 4.6 mm x 150 mm, 5 micron PV12s051546WT or equivalent.

Example 2 HPLC Procedure Substance 24

This method is an isocratic, normal phase HPLC method with peak detection at 220 nm wavelength.

Column temperature: 35-40 degrees C.
Injection volume: 20 μl to 100 μl.
Run time about 60 minutes.
Mobile phase of Heptane:Isopropyl Alcohol (95:5) is employed is used for the assay of Substance 24.
Condition column with Heptane: Isopropyl Alcohol 95:5 mobile phase at 1.0 ml/min until the blank injection baseline is stable. New column should be conditioned overnight at 0.2mL/min.
Examples of Column: Two PVA silica columns 4.6 mm x 150 mm, 5 micron PV12s051546WT or equivalent.
Flow rate 1.0 mL/min.

When a standard of a particular decomposition product is available, quantization of the impurity may be accomplished by standard procedures known in the art such as constructing a standard curve or by calculating a relative response factor (RRF). When a standard is not available a ratio of the area under the curve for the impurity to clevidipine can be used assuming a RRF previously calculated or if the RRF is not known an RRF of 1.0 is used to calculate the percent impurity

Calculation of percent impurity based on total peak area:

$$\frac{\text{impurity Peak Area}(100)}{(\text{total peak area of degradation products} + \text{H324/38 peak area (clevidipine peak area)})}$$

Calculation of percent impurity based on total peak area using H324/78 as the impurity example:

$$\frac{\text{H324/78 Peak Area(100)}}{\text{(total peak area of degradation products + H324/38 peak area (clevidipine peak area))}}$$

## Claims

1. A process for preparing a pharmaceutical composition that is stabilised against oxidation, wherein the composition is an emulsion for intravenous administration comprising:

   0.5 mg per mL of clevidipine or a pharmaceutically acceptable salt thereof,
   20% soybean oil,
   glycerin,
   purified egg yolk phospholipids,
   water,
   and sodium hydroxide,
   wherein the process comprises the steps of:

   (a) dispensing water for injection to a mix tank at 74 °C to 78 °C;
   (b) adding glycerin to the water and cooling the resulting aqueous phase to 60 °C to 70 °C;
   (c) dispensing soybean oil into a dissolving tank to form an oil phase;
   (d) mixing and heating the soybean oil to 70 °C to 82 °C;
   (e) adding clevidipine to the soybean oil mixture and heating to 78 °C to 82 °C;
   (f) adding egg yolk phospholipids to the mixture of clevidipine and soybean oil;
   (g) mixing the aqueous phase and the oil phase to form an emulsion;
   (h) adjusting the pH with 1N sodium hydroxide to a pH of 6.0 to 8.8; and
   (i) homogenising the emulsion at a pressure of 500/8000psi at a temperature of 50 °C to 55 °C;, and wherein oxidation of the composition is minimised by reducing the amount of light exposure during the manufacturing and storing processes, such that the amount of H324/78 having the following formula:

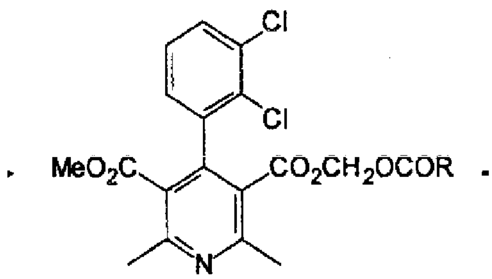

**H324/78**
R = $CH_2CH_2CH_3$

   is less than or equal to about 0.2% on a weight-to-weight basis to clevidipine.

2. A process according to claim 1, wherein the amount of H324/78 is such that the ratio of clevidipine, or pharmaceutically acceptable salts thereof, to H324/78 is greater than or equal to about 500 to 1 on a weight-to-weight basis.

3. A process according to claim 1 or claim 2, wherein the composition is stabilised by storage in a darkened container, for reducing exposure of the composition to light.

4. A process according to claim 3, wherein the darkened container is at least partially covered by secondary packaging.

5. A process according to any one of claims 1 to 4, wherein the oxidation degradation of clevidipine or a pharmaceutically

acceptable salt thereof is reduced to a rate of less than about 0.5% per day on a weight-to-weight basis.

**6.** A process according to claim 1, wherein the components are blanketed with an inert gas during the process of manufacturing and storing the emulsion, and wherein the composition is stored in a sealable and tinted, pigmented, colored, opaque or otherwise dark container that reduces the amount of light exposure.

**7.** A process according to claim 6, wherein the inert gas is nitrogen.

**8.** A process according to claim 6, wherein the container is at least partially covered by secondary packaging.

**Patentansprüche**

**1.** Verfahren zur Herstellung einer pharmazeutischen Zusammensetzung, die gegen Oxidation stabilisiert ist, wobei die Zusammensetzung eine Emulsion für intravenöse Verabreichung ist, umfassend:

0,5 mg/ml Clevidipin oder ein pharmazeutisch annehmbares Salz davon,
20 % Sojabohnenöl,
Glycerin,
gereinigte Eigelbphospholipide,
Wasser
und Natriumhydroxid,
wobei das Verfahren die folgenden Schritte umfasst:

(a) Dispensieren von Wasser zur Injektion an einen Mischtank bei 74°C bis 78°C;
(b) Zugeben von Glycerin zu dem Wasser und Abkühlen der resultierenden wässrigen Phase auf 60°C bis 70°C;
(c) Dispensieren von Sojabohnenöl in einen Auflösungstank, um eine Ölphase zu bilden;
(d) Mischen und Erwärmen des Sojabohnenöls auf 70°C bis 82°C;
(e) Zugeben von Clevidipin zu der Sojabohnenölmischung und Erwärmen auf 78°C bis 82°C;
(f) Zugeben von Eigelbphospholipiden zu der Mischung aus Clevidipin und Sojabohnenöl;
(g) Mischen der wässrigen Phase und der Ölphase, um eine Emulsion zu bilden;
(h) Einstellen des pH-Wertes mit 1N-Natriumhydroxid auf einen pH-Wert von 6,0-8,8; und
(i) Homogenisieren der Emulsion bei einem Druck von 500/8000 psi bei einer Temperatur von 50°C bis 55°C; und wobei Oxidation der Zusammensetzung minimiert wird, indem die Menge des Aussetzens an Licht während den Herstellungs- und Lagerungsverfahren reduziert wird, sodass die Menge an H324/78 mit der folgenden Formel:

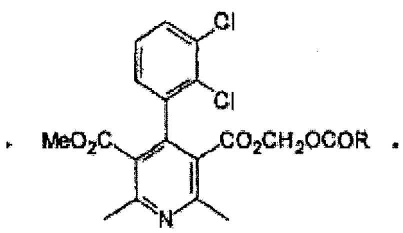

**H324/78**
R = $CH_2CH_2CH_3$

weniger als oder gleich etwa 0,2 % auf einer Gewicht-zu-Gewicht-Basis zu Clevidipin beträgt.

**2.** Verfahren gemäß Anspruch 1, wobei die Menge von H324/78 so ist, dass das Verhältnis von Clevidipin oder pharmazeutisch annehmbarer Salze davon zu H324/78 größer oder gleich etwa 500 bis 1 auf einer Gewicht-zu-Gewicht-Basis beträgt.

3. Verfahren gemäß Anspruch 1 oder Anspruch 2, wobei die Zusammensetzung durch Lagerung in einem abgedunkelten Behälter, um das Aussetzen der Zusammensetzung an Licht zu reduzieren, stabilisiert wird.

4. Verfahren gemäß Anspruch 3, wobei der abgedunkelte Behälter wenigstens teilweise durch eine Sekundärverpackung abgedeckt ist.

5. Verfahren gemäß einem der Ansprüche 1 bis 4, wobei der Oxidationsabbau von Clevidipin oder eines pharmazeutisch annehmbaren Salzes davon auf eine Rate von weniger als etwa 0,5 % pro Tag auf einer Gewicht-zu-Gewicht-Basis reduziert ist.

6. Verfahren gemäß Anspruch 1,
wobei die Komponenten während des Verfahrens der Herstellung und Lagerung der Emulsion mit einem Inertgas überlagert werden, und
wobei die Zusammensetzung in einem verschließbaren und getönten, pigmentierten, gefärbten, undurchsichtigen oder auf andere Weise dunklem Behälter, der die Menge des Aussetzen an Licht reduziert, gelagert wird.

7. Verfahren gemäß Anspruch 6, wobei das Inertgas Stickstoff ist.

8. Verfahren gemäß Anspruch 6, wobei der Behälter wenigstens teilweise durch eine Sekundärverpackung abgedeckt ist.

**Revendications**

1. Procédé pour la préparation d'une composition pharmaceutique qui est stabilisée contre l'oxydation, dans lequel la composition représente une émulsion destinée à une administration par voie intraveineuse, comprenant :

0,5 mg par ml de clévidipine ou d'un de ses sels pharmaceutiquement acceptables ;
20 % d'huile de soja ;
du glycérol ;
des phospholipides de jaune d'œuf purifiés ;
de l'eau ;
et de l'hydroxyde de sodium ;
dans lequel le procédé comprend les étapes dans lesquelles :

(a) on distribue de l'eau pour injection dans un réservoir de mélange à une température de 74 °C à 78 °C ;
(b) on ajoute du glycérol à l'eau et on refroidit la phase aqueuse résultante jusqu'à une température de 60 °C à 70 °C ;
(c) on distribue de l'huile de soja dans un réservoir de dissolution pour obtenir une phase huileuse ;
(d) on mélange et on chauffe l'huile de soja jusqu'à une température de 70 °C à 82 °C;
(e) on ajoute de la clévidipine au mélange à base d'huile de soja et on chauffe jusqu'à une température de 78 °C à 82 °C ;
(f) on ajoute des phospholipides de jaune d'œuf au mélange de clévidipine et d'huile de soja ;
(g) on mélange la phase aqueuse et la phase huileuse pour obtenir une émulsion ;
(h) on règle le pH avec de l'hydroxyde de sodium 1N afin d'obtenir un pH de 6,0 à 8,8 ; et
(i) on homogénéise l'émulsion sous une pression de 500/8000 psi à une température de 50 °C à 55 °C ; et

dans lequel on minimise l'oxydation de la composition en réduisant la quantité d'exposition à la lumière au cours des processus de confection et d'entreposage, d'une manière telle que la quantité de H324/78 répondant à la formule suivante :

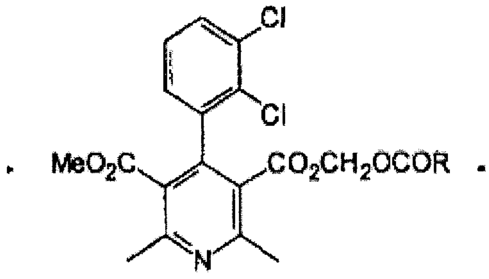

**H324/78**

$R = CH_2CH_2CH_3$

est inférieure ou égale à environ 0,2 % sur une base de poids à poids, par rapport à la clévidipine.

2. Procédé selon la revendication 1, dans lequel la quantité de H324/78 est telle que le rapport de la clévidipine ou d'un de ses sels pharmaceutiquement acceptables au H324/78 est supérieure ou égale à environ 500 à 1 sur une base de poids à poids.

3. Procédé selon la revendication 1 ou 2, dans lequel la composition est stabilisée par entreposage dans un récipient à l'abri de la lumière, afin de réduire l'exposition de la composition à la lumière.

4. Procédé selon la revendication 3, dans lequel le récipient à l'abri de la lumière est recouvert au moins en partie par une enveloppe secondaire.

5. Procédé selon l'une quelconque des revendications 1 à 4, dans lequel la dégradation par oxydation de la clévidipine ou d'un de ses sels pharmaceutiquement acceptables est réduite à un taux inférieur à environ 0,5 % par jour sur une base de poids à poids.

6. Procédé selon la revendication 1, dans lequel les composants sont recouverts d'un gaz inerte au cours des processus de confection et d'entreposage de l'émulsion, et dans lequel la composition est entreposée dans un récipient teinté, pigmentée, coloré, opaque ou conçu d'une autre manière pour être à l'abri de la lumière, qui réduit la quantité d'exposition à la lumière.

7. Procédé selon la revendication 6, dans lequel le gaz inerte est de l'azote.

8. Procédé selon la revendication 6, dans lequel le récipient est recouvert au moins en partie par une enveloppe secondaire.

Figure 1: Proposed degradation pathways of Clevidipine

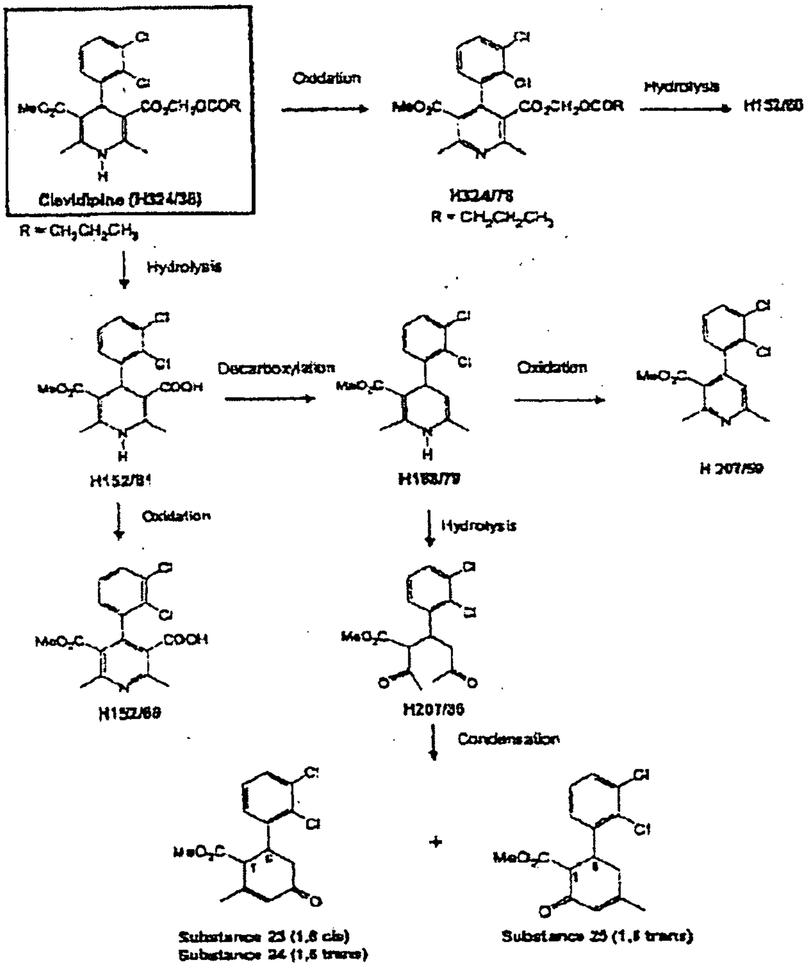

Figure 2A
Clevidipine Degradants Sample 2

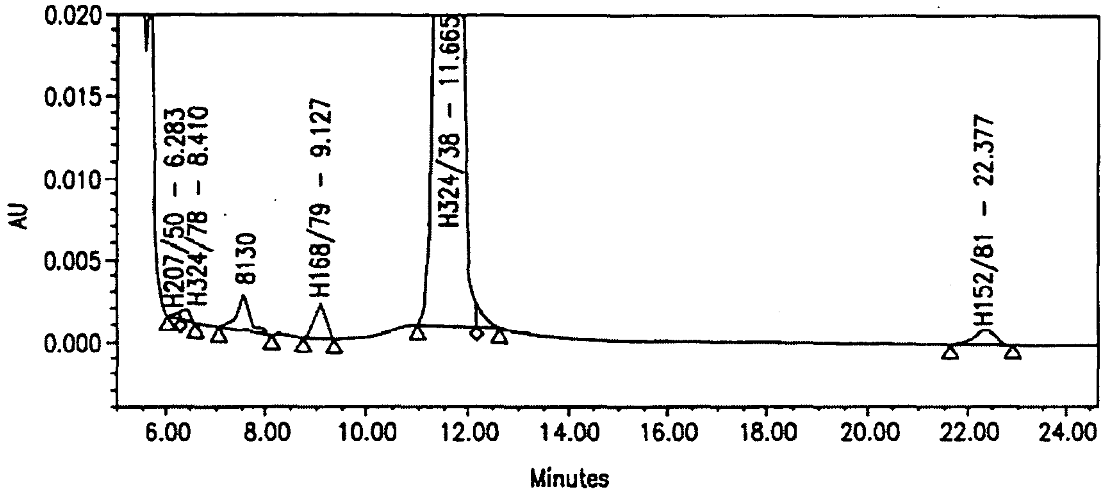


Figure 2B
Clevidipine Degradants Sample 3

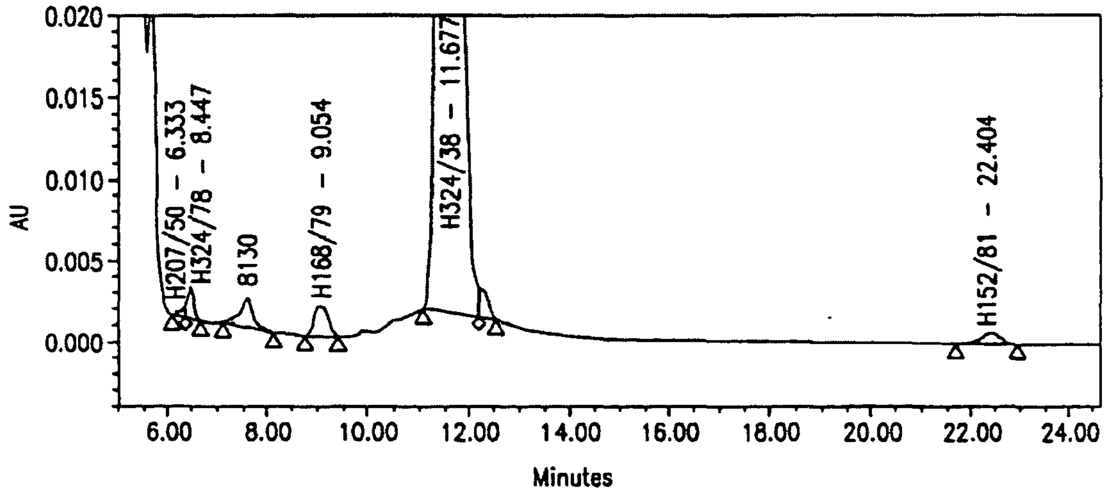

Figure 3
Overlay of HPLC Chromatograms of H324/78 Hydrolysis and Photostability Samples

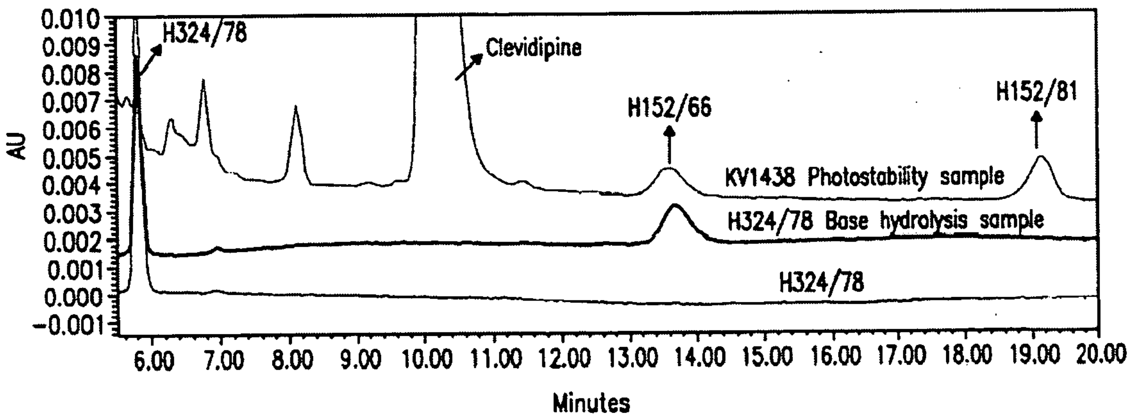

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5739152 A
- US 6350877 B